Europäisches Patentamt

European Patent Office

Office européen des brevets

(11) Numéro de publication: **0 261 727 B1**

# FASCICULE DE BREVET EUROPEEN

(45) Date de publication de fascicule du brevet: **02.12.92**  (51) Int. Cl.5: **C08F 4/64**, C08F 10/00

(21) Numéro de dépôt: **87201762.9**

(22) Date de dépôt: **15.09.87**

Le dossier contient des informations techniques présentées postérieurement au dépôt de la demande et ne figurant pas dans le présent fascicule.

(54) **Solide catalytique utilisable pour la polymérisation stéréospécifique des alpha-oléfines, procédé pour le préparer et procédé pour polymériser des alpha-oléfines en sa présence.**

(30) Priorité: **26.09.86 FR 8613649**

(43) Date de publication de la demande:
**30.03.88 Bulletin 88/13**

(45) Mention de la délivrance du brevet:
**02.12.92 Bulletin 92/49**

(84) Etats contractants désignés:
**AT BE CH DE ES FR GB GR IT LI LU NL SE**

(56) Documents cités:
**EP-A- 0 021 478**
**BE-A- 803 875**
**GB-A- 2 110 703**
**US-A- 4 463 102**

(73) Titulaire: **SOLVAY**
**Rue du Prince Albert, 33**
**B-1050 Bruxelles(BE)**

(72) Inventeur: **Fiasse, Paul**
**Rond Point de l'Etoile, 5**
**B-1050 Bruxelles(BE)**
Inventeur: **Bernard, Albert**
**Hippodroomlaan, 305**
**B-1960 Sterrebeek(BE)**

(74) Mandataire: **Husemann, Claude et al**
**SOLVAY Département de la Propriété Indus-**
**trielle 310, Rue de Ransbeek**
**B-1120 Bruxelles(BE)**

EP 0 261 727 B1

## Description

La présente invention concerne un solide catalytique utilisable pour la polymérisation stéréospécifique des alpha-oléfines, un procédé pour préparer ce solide et un procédé pour la polymérisation des alpha-oléfines en présence de ce solide.

Il est connu de polymériser stéréospécifiquement les alpha-oléfines telles que le propylène au moyen d'un système catalytique comprenant un constituant solide à base de trichlorure de titane et un activateur constitué d'un composé organométallique tel qu'un chlorure d'alkylaluminium.

Dans le brevet BE-A-780758 (SOLVAY & Cie), on a décrit des complexes catalytiques solides superactifs à base de $TiCl_3$ de porosité interne élevée, permettant d'obtenir des polymères du propylène de très bonne stéréorégularité.

Dans le brevet BE-A-803875 (SOLVAY & Cie), on a décrit un traitement de préactivation de ces complexes catalytiques solides superactifs qui permet de les stocker sous hexane pendant de longues périodes sans qu'ils perdent leurs qualités. Le préactivateur utilisé peut être choisi parmi les composés organoaluminiques et, notamment, suivant la formule (I) qui précise leur nature, parmi les hydrocarbylhydrocarbyloxyhalogénures d'aluminium. En pratique, toutefois, il est fait usage de chlorure de diéthylaluminium, de sesquichlorure d'éthylaluminium, de dichlorure d'éthylaluminium et de triéthylaluminium.

La stéréospécificité de ces complexes catalytiques n'est malheureusement pas suffisante dans toutes les conditions de polymérisation dans lesquelles on peut être amené à les utiliser, et notamment pas aux températures relativement élevées auxquelles on effectue fréquemment la polymérisation du propylène en phase gazeuse. En effet, lorsqu'on effectue la polymérisation à des températures relativement basses, on observe une diminution importante de la productivité catalytique.

On a tenté de remédier à cet inconvénient en effectuant la polymérisation du propylène en présence de systèmes catalytiques comprenant les complexes catalytiques solides superactifs mentionnés plus haut, modifiés par l'introduction, dans le milieu de polymérisation, d'un terconstituant qui est généralement un composé électrodonneur (base de Lewis). Un très grand nombre de composés électrodonneurs de natures diverses ont déjà été proposés à titre de terconstituants susceptibles d'augmenter la stéréospécificité de ces systèmes catalytiques (voir par exemple le brevet BE-A-822941 (ICI)). Parmi ce très grand nombre de composés électrodonneurs utilisables à ce titre, on a proposé notamment certains composés phénoliques (demande de brevet EP-A-0036549 (BASF)) et certains composés hydroxyaromatiques (brevet US-A-4478989 (SHELL OIL)). L'amélioration de stéréospécificité obtenue grâce à l'introduction de pareils composés électrodonneurs dans le milieu de polymérisation n'est toutefois notable que lorsque la quantité de composé électrodonneur est relativement élevée (le poids de composé électrodonneur est généralement au moins égal au poids de complexe catalytique solide présent dans le milieu et souvent très supérieur). On constate alors des effets secondaires néfastes tels une diminution inacceptable de la productivité catalytique et l'apparition de colorations parasites dans le polymère recueilli sans parler des complications que peut entraîner l'obligation d'épurer le polymère en résidus de terconstituant.

Dans le document GB-A 2110703, on décrit un procédé de polymérisation d'alpha-oléfines en présence d'un système catalytique comprenant du trichlorure de titane, un aluminium alkyle et un composé phénolique antioxydant et on cherche expressément à éviter une cristallisation intempestive des composés du système catalytique pendant le transfert de celui-ci vers le réacteur de polymérisation. A cet effet, on commence par traiter le trichlorure de titane avec un milieu de préactivation obtenu en faisant réagir le composé phénolique avec une quantité déficitaire du composé aluminique, de sorte que le système catalytique obtenu soit inactif. Celui-ci est ensuite introduit tel quel, à l'état d'une suspension liquide (slurry) dans un réacteur où on introduit en outre le monomère et le solde du composé aluminique nécessaire pour rendre le système catalytique actif.

Dans le document EP-A 0021478, on décrit un procédé de polymérisation d'alpha-oléfines, en présence d'un système catalytique mettant en oeuvre du trichlorure de titane et un activateur. Selon ce procédé connu, on prépare d'abord l'activateur en faisant réagir un composé organoaluminique halogéné, éventuellement un composé organoaluminique non halogéné et un composé hydroxyaromatique comprenant deux groupes sec- ou ter-alkyle en position ortho par rapport au groupe hydroxyle. L'activateur ainsi préparé est introduit dans le réacteur de polymérisation dans lequel on introduit ensuite le trichlorure de titane et le monomère. Le système catalytique est ainsi fabriqué in situ dans le réacteur, au moment de la polymérisation.

La présente invention vise à fournir un solide catalytique dont la stéréospécificité est très élevée sans qu'il soit nécessaire d'introduire un terconstituant dans le milieu de polymérisation.

La présente invention concerne à cet effet des solides catalytiques à base de trichlorure de titane complexé, utilisables pour la polymérisation stéréospécifique des alphaoléfines, préactivés par mise en

2

contact avec un préactivateur organoaluminique, puis séparation du milieu de préactivation, le préactivateur comprenant le produit de la réaction d'un composé organoaluminique (a) et d'un composé (b) choisi parmi les composés hydroxyaromatiques dont le groupe hydroxyle est stériquement bloqué.

Les solides à base de trichlorure de titane complexé utilisés comme précurseurs pour préparer les solides catalytiques préactivés selon la présente invention peuvent être obtenus par n'importe quel procédé connu. On préfère généralement mettre en oeuvre des solides obtenus par des procédés impliquant une réduction initiale du tétrachlorure de titane. Cette réduction peut être opérée à l'intervention d'hydrogène ou de métaux tels que le magnésium et de préférence l'aluminium. Les meilleurs résultats sont obtenus au départ de solides formés par réduction du tétrachlorure de titane par un réducteur organométallique. Celui-ci peut être par exemple un réducteur organomagnésien. Toutefois, les meilleurs résultats sont obtenus avec des réducteurs organoaluminiques (1).

Les réducteurs organoaluminiques (1) utilisables de préférence sont les composés qui contiennent au moins un radical hydrocarboné fixé directement sur l'atome d'aluminium. Des exemples de composés de ce type sont les mono-, di- et trialkylaluminiums dont les radicaux alkyles contiennent de 1 à 12, et de préférence de 1 à 6 atomes de carbone, tels le triéthylaluminium, les isoprénylaluminiums, l'hydrure de diisobutylaluminium et l'éthoxydiéthylaluminium. Avec les composés de ce type, les meilleurs résultats sont obtenus avec les chlorures de dialkylaluminiums et, en particulier, avec le chlorure de diéthylaluminium.

Pour obtenir les solides à base de trichlorure de titane complexé (appelés ci-après "précurseurs") utilisés pour préparer les solides catalytiques préactivés selon la présente invention, on soumet les solides réduits mentionnés ci-dessus à un traitement au moyen d'au moins un agent complexant qui est choisi en général parmi les composés organiques comprenant un ou plusieurs atomes ou groupements présentant une ou plusieurs paires d'électrons libres susceptibles d'assurer la coordination avec les atomes de titane ou d'aluminium présents dans les halogénures de titane ou d'aluminium. De préférence, l'agent complexant est choisi dans la famille des éthers aliphatiques, et plus particulièrement parmi ceux dont les radicaux aliphatiques comprennent de 2 à 8 atomes de carbone, de préférence 4 à 6 atomes de carbone. Un exemple typique d'éther aliphatique donnant de très bons résultats est l'éther diisoamylique.

Ces traitements au moyen d'agents complexants, propres à stabiliser ou à améliorer la productivité et/ou la stéréospécificité des solides catalytiques sont bien connus et ont été amplement décrits dans la littérature.

Ainsi, pour la préparation du précurseur, le traitement au moyen de l'agent complexant peut consister en un broyage du solide réduit en présence de l'agent complexant. Il peut consister en un traitement thermique du solide réduit en présence de l'agent complexant. Il peut aussi consister en des lavages extractifs du solide réduit, en présence de solvants mixtes comprenant un composé hydrocarboné liquide et un solvant auxiliaire polaire, par example un éther. On peut aussi effectuer la réduction du tétrachlorure de titane avec le réducteur organoaluminique (1), en présence de l'agent complexant, par exemple en ajoutant au tétrachlorure de titane une solution hydrocarbonée du produit de la réaction de l'agent complexant avec ce réducteur, et soumettre ensuite le solide réduit ainsi obtenu à un traitement thermique en l'absence d'agent complexant ou en présence d'une nouvelle quantité d'agent complexant, identique ou différent du précédent. On peut aussi effectuer le traitement au moyen de l'agent complexant avec une quantité de ce dernier suffisante pour former une solution homogène de solide à base de trichlorure de titane et reprécipiter pa r chauffage le solide ainsi dissous

Pour la préparation du précurseur, le traitement au moyen de l'agent complexant peut être combiné avec ou suivi d'un traitement d'activation. Ces traitements d'activation sont également bien connus et ont également été décrits dans la littérature. Ils sont effectués en général au moyen d'au moins un agent choisi parmi les composés halogénés inorganiques, les composés halogénés organiques, les composés interhalogénés et les halogènes. Parmi ces agents, on peut citer :

- à titre de composés halogénés inorganiques, les halogénures de métaux et de non métaux, tels que les halogénures de titane et de silicium par exemple;
- à titre de composés halogénés organiques, les hydrocarbures halogénés tels que les alkanes halogénés et les tétrahalogénures de carbone par exemple;
- à titre de composés interhalogénés, les chlorure et bromure d'iode par exemple;
- à titre d'halogène, le chlore, le brome et l'iode.

Des exemples d'agents convenant très bien pour le traitement d'activation sont le tétrachlorure de titane, le tétrachlorure de silicium, l'iodobutane, le monochloréthane, l'hexachloréthane, le chlorméthylbenzène, le tétrachlorure de carbone, le chlorure d'iode et l'iode. Les meilleurs résultats ont été obtenus avec le tétrachlorure de titane.

La forme physique sous laquelle se trouvent les agents complexants et les agents utilisés pour l'éventuel traitement d'activation n'est pas critique pour la préparation du précurseur. Ces agents peuvent

EP 0 261 727 B1

être mis en oeuvre sous forme gazeuse, ou sous forme liquide, cette dernière étant la forme la plus usuelle sous laquelle ils se présentent dans les conditions habituelles de température et de pression. On peut aussi effectuer le traitement au moyen de l'agent complexant et l'éventuel traitement d'activation en présence d'un diluant hydrocarboné inerte, généralement choisi parmi les hydrocarbures aliphatiques, cycloaliphatiques et aromatiques liquides, tels que les alkanes et isoalkanes liquides et le benzène.

Des détails relatifs aux conditions opératoires des traitements de complexation et d'activation les plus courants peuvent être trouvés notamment dans le brevet BE-A-864708 (SUMITOMO CHEMICAL COMPANY LTD), dans le brevet US-A-4295991 (EXXON RESEARCH AND ENGINEERING CO) et dans les documents cités dans ce dernier.

A un moment quelconque de sa préparation, après l'étape de réduction ou de complexation, ou après l'éventuelle étape d'activation, mais de préférence après l'étape de réduction, le précurseur peut être soumis à un traitement visant à diminuer la friabilité de ses particules constitutives. Ce traitement, appelé "prépolymérisation", consiste à mettre le solide en contact avec une alpha- monooléfine inférieure, telle que l'éthylène ou, mieux, le propylène, dans des conditions polymérisantes de manière à obtenir un solide contenant en général entre 5 et 500 % en poids environ d'alpha-monooléfine "prépolymérisée". Cette "prépolymérisation" peut s'effectuer avantageusement au sein d'une suspension du solide dans le diluant hydrocarboné inerte tel que défini plus haut pendant une durée suffisante pour obtenir la quantité souhaitée d'alpha-monooléfine prépolymérisée sur le solide. Le précurseur obtenu selon cette variante est moins friable et permet d'obtenir des polymères de bonne morphologie même lorsqu'on polymérise à température relativement élevée.

Pour sa transformation en solide catalytique préactivé, décrite plus loin, le précurseur peut être mis en oeuvre tel quel, c'est-à-dire sans le séparer du milieu dans lequel il a été préparé, ou, de préférence, après séparation et éventuel lavage par un diluant hydrocarboné inerte tel que défini plus haut.

Une méthode de préparation préférée de solides à base de trichlorure de titane complexé utilisables comme précurseurs pour préparer les solides catalytiques préactivés selon la présente invention a été décrite dans le brevet BE-A-780758. Cette méthode c omprend la réduction du tétrachlorure de titane au moyen d'un réducteur organoaluminique (1) qui, en l'occurrence, est de préférence un chlorure de dialkylaluminium dont les chaînes alkyles comprennent de 2 à 6 atomes de carbone, dans des conditions douces. Après un éventuel traitement thermique du solide réduit ainsi obtenu, on soumet ce dernier à un traitement au moyen d'un agent complexant tel que défini plus haut. Enfin, on opère un traitement au moyen de tétrachlorure de titane et on sépare le solide à base de trichlorure de titane complexé ainsi formé que l'on lave éventuellement au moyen d'un diluant hydrocarboné inerte tel que défini plus haut, choisi de préférence parmi les hydrocarbures aliphatiques liquides comprenant de 3 à 12 atomes de carbone et qui est d'ailleurs le diluant qui peut être utilisé tout au long de la préparation dudit solide.

La méthode de préparation préférée définie dans le paragraphe précédent conduit à des particules de solide à base de trichlorure de titane complexé qui sont également décrites dans le brevet BE-A-780758. Ces particules sont sphériques et ont en général un diamètre compris entre 5 et 100 microns et le plus souvent entre 10 et 50 microns. Elles sont constituées d'un aggloméat de microparticules également sphériques qui ont un diamètre compris entre 0,05 et 1 micron, le plus souvent entre 0,1 et 0,3 micron et qui sont extrêmement poreuses. Il en résulte que les particules présentent une surface spécifique supérieure à 75 $m^2/g$ et se situant le plus souvent entre 100 et 250 $m^2/g$ et une porosité totale supérieure à 0,15 $cm^3/g$ et comprise la plupart du temps entre 0,20 et 0,35 $cm^3/g$. La porosité interne des microparticules constitue la contribution la plus importante à cette porosité totale des particules, comme l'atteste la valeur élévée du volume poreux correspondant aux pores de moins de 200 Å de diamètre, qui est supérieur à 0,11 $cm^3/g$ et la plupart du temps compris entre 0,16 et 0,31 $cm^3/g$.

Les solides à base de trichlorure de titane complexé (précurseurs) obtenus selon la méthode de préparation décrite dans le brevet BE-A-780758 en choisissant les conditions opératoires préférées répondent à la formule :

$$TiCl_3 . (AlR''Cl_2)_x . C_y$$

où R'' est un radical alkyle comprenant de 2 à 6 atomes de carbone, C est un agent complexant tel que défini plus haut, x est un nombre quelconque inférieur à 0,20 et y un nombre quelconque supérieur à 0,009 et en général inférieur à 0,20.

A titre de variante de cette méthode de préparation, on peut citer celle, mentionnée plus haut, consistant à "prépolymériser" le solide réduit, après l'éventuel traitement thermique et avant le traitement au moyen de l'agent complexant, avec une alpha-monooléfine inférieure (propylène) dans des conditions polymérisantes. Cette "prépolymérisation" s'effectue au sein d'une suspension du solide réduit dans le

4

diluant hydrocarboné inerte tel que défini plus haut, entre 20 et 80″C environ, pendant une durée comprise généralement entre 1 minute et 1 heure.

Selon l'invention, le précurseur, préparé comme décrit ci-avant, est mis en contact avec un préactivateur organoaluminique qui comprend le produit de la réaction d'un composé organoaluminique (a) et d'un composé (b) choisi parmi les composés hydroxyaromatiques dont le groupe hydroxyle est stériquement bloqué.

Le composé organoaluminique (a) est généralement choisi parmi les composés de formule :

$$Al \; R_n \; X_{3-n}$$

dans laquelle R représente des radicaux hydrocarbonés identiques on différents contenant de 1 à 18 atomes de carbone, choisis en particulier parmi les radicaux alkyle, aryle, arylalkyle, alkylaryle, cycloalkyle, alkoxy et aryloxy; X est un halogène; n est un nombre tel que $0 < n \leq 3$.

Dans la formule ci-dessus, R est de préférence un radical alkyle, linéaire ou branché, contenant de 2 à 8 atomes de carbone, X est de préférence le chlore et n est de nce tel que $1 \leq n \leq 3$.

A titre d'exemples de composés (a), on peut citer : les trialkylaluminiums, tels que les triméthyl-, triéthyl-, tri-n-propyl, tri-n-butyl-, tri-i-butyl-, tri-n-hexyl-, tri-i-hexyl-, tri-2-méthylpentyl- et tri-n-octylaluminium; les monohalogénures de dialkylaluminium, tels que les monochlorures de diéthyl-, di-n-propyl- et di-i-butylaluminium, les monofluorures, monobromures et monoiodures d'éthylaluminium; les di-et sesquihalogénures d'alkylaluminium, tels que les sesquichlorures de méthyl- et d'éthylaluminium et les dichlorures d'éthyl- et d'isobutylaluminium; les halogénures d'alkoxyaluminium, tels que les dichlorures de méthoxy- et d'isobutoxyaluminium; les alkoxyalkylaluminiums, tels que le monoéthoxydiéthylaluminium, le diéthoxymonoéthylaluminium et le dihexanoxymono-n-hexylaluminium.

De très bons résultats ont été obtenus avec les trialkylaluminiums et les chlorures de dialkylaluminium, en particulier avec le triéthylaluminium et le monochlorure de diéthylaluminium.

Le composé (b) est choisi parmi les composés hydroxyaromatiques dont le groupe hydroxyle est stériquement bloqué. Par composé hydroxyaromatique dont le groupe hydroxyle est stériquement bloqué, on entend désigner tous les composés hydroxyaromatiques qui comprennent un radical alkyle secondaire ou tertiaire dans les deux positions ortho par rapport au groupe hydroxyle.

Le composé (b) est généralement choisi parmi les hydroxyarylènes mono- ou polycycliques substitués comme indiqué ci-dessus, en particulier parmi les hydroxybenzènes, les hydroxynaphtalènes, les hydroxyanthracènes et les hydroxyphénanthrènes ainsi substitués, et dont les noyaux aromatiques peuvent encore porter d'autres substituants.

A titre d'exemples de composés (b), on peut citer :

- les monophénols monocycliques di-tert- et di-sec-alkylés dans les positions ortho par rapport au groupe hydroxyle, tels que le 2,6-di-tert-butylphénol, le 2,6-di-tert-butyl-4-méthylphénol, le 2,6-di-tert-décyl-4-méthoxy-phénol, le 2,6-di-tert-butyl-4-isopropylphénol, le 2,6-dicyclohexyl-4-méthylphénol, le 2,6-diisopropyl-4-méthoxyphénol et le 2,6-di-t-butyl-4-sec-butylphénol;
- les monoesters de l'acide 3-(3′,5′-di-tert-butyl-4′-hydroxyphényl)propionique, tels que les 3-(3′,5′-di-tert-butyl-4'-hydroxyphényl)propionates de méthyle, d'éthyle, de n-propyle, de n-butyle, de n-octyle, de n-dodécyle et de n-octadécyle;
- les polyphénols di-tert-alkylés dans les positions ortho par rapport aux groupes hydroxyles, tels que le 2,2-bis(2,6-di-tert-butyl-hydroxy-phényl)propane, le bis(3,5-di-tert-butyl-4-hydroxybenzyl)méthane, le 4,4′-méthylène-bis(2,6-di-tert-butyl)phénol, le 2,2′-méthylène-bis(4-éthyl-6-tert-butyl)phénol, le 1,3,5-triméthyl-2,4,6-bis(3,5-di-tert-butyl-4-hydroxybenzyl)benzène et le tris(2,6-di-tert-hexylhydroxy-phényl)-benzène;
- les polyesters de l'acide 3-(3′,5′-di-tert-butyl-4'-hydroxylphényl)propionique, tels que le tétrakis-[méthylène-3-(3′,5′-di-tert-butyl-4′-hydroxyphényl) ropionate]méthane;
- les monophénols polycycliques di-tert- et di-sec-alkylés dans les positions ortho par rapport au groupe hydroxyle, tels que le 1,3-di-tert-butyl-2-hydroxyanthracène, le 1,3-di-tert-hexyl-2-hydroxyphénanthrène, le 2,8-di-tert-butyl-hydroxynaphtalène, le 1,3-di-tert-hexyl-2-hydroxynaphtalène et le 1,3-diisoamyl-2-hydroxynaphtalène.

De très bons résultats ont été obtenus avec les monophénols monocycliques di-tert-alkylés, en particulier avec le 2,6-di-tert-butyl-4-méthylphénol et avec les monoesters de l'acide 3-(3′,5′-di-tert-butyl-4'-hydroxyphényl)propionique, en particulier avec le 3-(3′,5′-di-tert-butyl-4'-hydroxyphényl) propionate de n-octadécyle, l'utilisation de ce dernier conférant une excellente stéréospécificité aux solides catalytiques préparés à son intervention.

Les conditons générales dans lesquelles o contact le composé (a) avec le composé (b) ne sont pas critiques pour autant qu'elles induisent une réaction chimique entre ces composés. En général, on opère en phase liquide, par example en mélangeant le composé (b) et le composé (a) en l'absence de diluant liquide, le composé (a) étant souvent liquide dans des conditions normales de température et de pression. On peut aussi opérer en présence d'un diluant hydrocarboné inerte tel que défini plus haut.

Le rapport molaire selon lequel le composé (a) et le composé (b) sont mis en présence peut varier dans une large mesure. En général, on met en oeuvre de 50 à 0,1 moles de composé (a) par mole de composé (b); de préférence, la quantité de composé (a) mise en oeuvre est comprise entre 15 et 0,5 moles par mole de composé (b); de très bons résultats ont été enregistrés pour des rapports molaires du composé (a) au composé (b) compris entre 3 et 1 environ.

Les composés (a) et (b) peuvent être mis en contact à des températures généralement comprises entre environ 0 et 90°C, de préférence à une température voisine de la température ambiante (25°C) et leur mélange maintenu pendant un temps, suffisant pour qu'ils puissent réagir chimiquement entre eux, généralement compris entre 5 minutes et 5 heures. Cette réaction s'accompagne le plus souvent d'un dégagement gazeux qui permet d'en apprécier l'avancement.

La structure chimique exacte du produit de la réaction des composés (a) et (b) n'est pas connue avec certitude. Il est toutefois pratiquement certain que ce produit répond, au moins partiellement, à la formule empirique :

$$R_p \, Al(OR')_q \, X_{3-(p+q)}$$

dans laquelle :
- R qui a la signification donnée ci-avant, représente le(les) même(s) radical(radicaux) hydrocarboné(s) que celui(ceux) contenu(s) dans le composé organoaluminique (a);
- OR' représente le groupe aryloxy dérivé du composé (b);
- X est un halogène;
- p est un nombre tel que $0 < p < 3$, de préférence tel que $0,1 \leq p \leq 2,5$;
- q est un nombre tel que $0 < q < 2$, de préférence tel que $0,5 \leq q \leq 1,5$;
- la somme $(p+q)$ étant telle que $0 < (p+q) \leq 3$.

Selon l'invention, on met le préactivateur organoaluminique, obtenu comme décrit ci-dessus, en contact avec le précurseur.

Les conditions opératoires de la mise en contact du préactivateur avec le précurseur ne sont pas critiques pour autant qu'elles entraînent une fixation au moins partielle du préactivateur sur ledit précurseur.

A cette réserve près, cette mise en contact peut se faire par n'importe quel procédé connu. On peut par exemple effectuer cette mise en contact par broyage du précurseur imprégné par une phase liquide contenant le préactivateur.

Le plus souvent, le préactivateur est mis en oeuvre sous la forme d'une solution, dans le diluant hydrocarboné inerte optionnellement utilisé pour sa préparation :
(1) du produit de la réaction du composé (a) avec le composé (b) éventuellement accompagné
(2) de l'excès de composé (a) ou de composé (b) mis en oeuvre et non réagi.

Dans ce cas, on préfère introduire la solution de préactivateur contenant l'ingrédient (1) et l'éventuel ingrédient (2) dans une suspension du précurseur dans ce même diluant hydrocarboné. Cette suspension est alors maintenue en général à une température comprise entre 0°C et la température d'ébullition normale du diluant hydrocarboné inerte dans lequel le préactivateur a été dissous, de préférence entre 20 et 40°C environ, pour une durée généralement comprise entre environ 5 et environ 120 minutes, de préférence entre 15 et 90 minutes. Les quantités respectives de précurseur et de préactivateur mises en oeuvre sont telles que le rapport molaire entre la quantité initiale totale de composé (a) utilisé et la quantité de $TiCl_3$ présent dans le précurseur est généralement compris entre $10^{-3}$ et 10, de préférence entre $10^{-2}$ et 1. De très bons résultats ont été obtenus lorsque le rapport molaire défini ci-dessus est compris entre 0,05 et 0,5.

A la fin de cette étape de préactivation, le solide catalytique ainsi préactivé est séparé du milieu de préactivation et lavé pour éliminer les résidus de préactivateur non fixé, de préférence au moyen d'un diluant hydrocarboné inerte de même nature que ceux optionnellement utilisés pour préparer le précurseur et la solution de préactivateur.

Le solide catalytique préactivé, séparé et lavé, peut être ensuite éventuellement séché. Il peut par exemple être séché jusqu'à ce que sa teneur en diluant hydrocarboné liquide résiduaire soit inférieure à 1 % en poids, de préférence inférieure à 0,5 % en poids par rapport au poids du trichlorure de titane qu'il contient, selon les conditions opératoires décrites dans le brevet BE-A-846911 (SOLVAY & Cie).

Le solide catalytique préactivé ainsi obtenu comprend toujours une certaine quantité de préactivateur fixée sur le solide et que l'on ne peut dissocier de ce dernier par des méthodes de séparation purement physiques. Cette quantité de préactivateur est comprise en général entre 5 et 500 g par kg de TiCl$_3$ présent dans le solide, de préférence entre 50 et 300 g/kg. Dès lors, le solide catalytique préactivé suivant l'invention contient moins de TiCl$_3$ par unité de poids que le solide utilisé comme précurseur pour le préparer. Quoique le solide catalytique préactivé contienne en général au moins 50 % en poids de TiCl$_3$ par rapport au poids total, il en contient rarement plus de 80 % environ.

La morphologie externe des particules de solide catalytique préactivé selon l'invention ne se distingue pas de celle des particules de précurseur utilisé pour les préparer. C'est ainsi que, lorsqu'elles sont préparées au départ de particules sphériques constituées d'un aggloméral de microparticules sphériques poreuses, elles ont sensiblement la même structure, les mêmes dimensions et les mêmes formes que les particules de départ. Toutefois, les particules de solide catalytique préactivé sont moins poreuses, c'est-à-dire qu'elles ne se caractérisent plus par la surface spécifique élevée associée au volume poreux élevé caractérisant les particules de précurseur.

Après avoir été lavés et éventuellement séchés, les solides catalytiques préactivés selon l'invention peuvent être immédiatement remis en contact avec un diluant hydrocarboné inerte tel que ceux qui ont été définis plus haut et qui sont aussi utilisables comme diluants dans le polymérisation en suspension. Les solides catalytiques préactivés selon l'invention peuvent aussi être soumis à un traitement de "prépolymérisation" tel que décrit plus haut en rapport avec le précurseur. Ils peuvent être stockés sous hexane ou sous forme sèche, de préférence à froid, pendant des périodes longues sans perdre leurs qualités.

Pour la polymérisation, le solide catalytique préactivé selon l'invention est utilisé conjointement avec un activateur choisi parmi les composés organométalliques de métaux des groupes Ia, IIa, IIb et IIIb du Tableau Périodique (version publiée dans Kirk-Othmer Encyclopedia of Chemical Technology, 2nd completely revised edition, volume 8, 1965, page 94) et de préférence parmi les composés de formule :

$$\text{Al R'''}_m\ Y_{3\text{-}m}$$

où

- R''' est un radical hydrocarboné contenant de 1 à 18 atomes de carbone et de préférence de 1 à 12 atomes de carbone choisi parmi les radicaux alkyle, aryle, arylalkyle, alkylaryle et cycloalkyle; les meilleurs résultats sont obtenus lorsque R''' est choisi parmi les radicaux alkyle contenant de 2 à 6 atomes de carbone;
- Y est un halogène choisi parmi le fluor, le chlore, le brome et l'iode; les meilleurs résultats sont obtenus lorsque Y est le chlore;
- m est un nombre quelconque tel que $0 < m \leq 3$ et de préférence tel que $1,5 \leq m \leq 2,5$; les meilleurs résultats sont obtenus lorsque m est égal à 2.

Le chlorure de diéthylaluminium (DEAC) assure une activité et une stéréospécificité maximales du système catalytique.

Les systèmes catalytiques ainsi définis s'appliquent à la polymérisation des oléfines à insaturation terminale dont la molécule contient de 2 à 18 et de préférence de 2 à 6 atomes de carbone telles que l'éthylène, le propylène, le butène-1, le pentène-1, les méthylbutène-1, l'hexène-1, les 3- et 4-méthylpentènes-1 et le vinylcyclohexène. Ils sont particulièrement intéressants pour la polymérisation stéréospécifique du propylène, du butène-1 et du 4-méthylpentène-1 en polymères cristallins, fortement isotactiques. Ils s'appliquent également à la copolymérisation de ces alpha-oléfines entre elles ainsi qu'avec des dioléfines comprenant de 4 à 18 atomes de carbone. De préférence, les dioléfines sont des dioléfines aliphatiques non conjuguées telles que l'hexadiène-1,4, des dioléfines monocycliques non conjuguées telles que le 4-vinylcyclohexène, des dioléfines alicycliques ayant un pont endocyclique telles que le dicyclopentadiène, le méthylène- et l'éthylènenorbornène et des dioléfines aliphatiques conjuguées telles que le butadiène ou l'isoprène.

Ils s'appliquent encore à la fabrication de copolymères appelés à blocs qui sont constitués à partir d'alpha-oléfines et de dioléfines. Ces copolymères à blocs consistent en des successions de segments de chaîne à longueurs variables; chaque segment consiste en un homopolymère d'une alpha-oléfine ou en un copolymère statistique comprenant une alpha-oléfine et au moins un comonomère choisi parmi les alpha-oléfines et les dioléfines. Les alpha-oléfines et les dioléfines sont choisies parmi celles mentionnées ci-dessus.

Les solides catalytiques préactivés selon l'invention conviennent particulièrement bien pour la fabrication d'homopolymères du propylène et de copolymères contenant au total au moins 50 % en poids de propylène et de préférence 75 % en poids de propylène.

La polymérisation peut être effectuée selon n'importe quel procédé connu : en solution ou en suspension dans un solvant ou und diluant hydrocarboné inerte, tel que ceux définis en rapport avec la préparation du solide catalytique et qui est de préférence choisi parmi le butane, le pentane, l'hexane, l'heptane, le cyclohexane, le méthylcyclohexane ou leurs mélanges. On peut également opérer la polymérisation dans le monomère ou un des monomères maintenu à l'état liquide ou encore en phase gazeuse. L'utilisation des solides catalytiques préactivés selon l'invention est très avantageuse pour la polymérisation en phase gazeuse. Dans la mesure, en effet, où l'apparition de sous-produits amorphes et collants est particulièrement nuisible dans ce type de polymérisation, dont la technologie n'en permet pas l'élimination, l'utilisation de systèmes catalytiques très stéréospécifiques y est spécialement intéressante.

La température de polymérisation est choisie généralement entre 20 et 200°C et de préférence entre 50 et 90°C, les meilleurs résultats étant obtenus entre 65 et 85°C. La pression est choisie généralement entre la pression atmosphérique et 50 atmosphères et de préférence entre 10 et 30 atmosphères. Cette pression est bien entendu fonction de la température utilisée.

La polymérisation peut être effectuée en continu ou en discontinu.

La préparation des copolymères dits à blocs peut se faire également selon des procédés connus. On préfère utiliser un procédé en deux étapes consistant à polymériser une alpha-oléfine, généralement le propylène, selon la méthode décrite précédemment pour l'homopolymérisation. Ensuite, on polymérise l'autre alpha-oléfine et/ou dioléfine, généralement l'éthylène, en présence de la chaîne homopolymère encore active. Cette seconde polymérisation peut se faire après avoir enlevé complètement ou partiellement le monomère n'ayant pas réagi au cours de la première étape.

Le composé organométallique et le solide catalytique préactivé uvent être ajoutés séparément au milieu de polymérisation. On peut également les mettre en contact, à une température comprise entre -40 et 80°C, pendant une durée qui est dépendante de cette température et qui peut aller de une heure à plusieurs jours, avant de les introduire dans le réacteur de polymérisation.

La quantité totale de composé organométallique mise en oeuvre n'est pas critique; elle est en général supérieure à 0,1 mmole par litre de diluant, de monomère liquide, ou de volume de réacteur, de préférence supérieure à 0,5 mmole par litre.

La quantité de solide catalytique préactivé mise en oeuvre est déterminée en fonction de sa teneur en $TiCl_3$. Elle est choisie en général de manière que la concentration du milieu de polymérisation soit supérieure à 0,01 mmole de $TiCl_3$ par litre de diluant, de monomère liquide ou de volume de réacteur et de préférence supérieure à 0,05 mmole par litre.

Le rapport des quantités de composé organométallique et de solide catalytique préactivé n'est pas critique non plus. On le choisit généralement de manière que le rapport molaire composé organométallique/$TiCl_3$ présent dans le solide soit compris entre 0,5 et 20 et de préférence entre 1 et 15. Les meilleurs résultats sont obtenus lorsque le rapport molaire est compris entre 2 et 12.

Le poids moléculaire des polymères fabriqués selon le procédé de l'invention peut être réglé par l'addition au milieu de polymérisation d'un ou plusieurs agents de réglage du poids moléculaire comme l'hydrogène, le diéthylzinc, les alcools, les éthers et les halogénures d'alkyle.

La stéréospécificité des solides catalytiques préactivés selon l'invention est plus élevée que celle des complexes catalytiques décrits dans le brevet BE-A-780758, lorsqu'ils sont préparés à partir de ces derniers. De plus, cette stéréospécificité reste inaltérée pendant de très longues périodes, même lorsque les solides catalytiques préactivés sont stockés à température relativement élevée. Il n'est donc plus nécessaire, lorsqu'on les utilise, d'ajouter au milieu de polymérisation un terconstituant conventionnellement connu pour améliorer cette stéréospécificité, tel qu'un éther ou un ester, par exemple. Il va évidemment de soi que pareille addition d'un terconstituant au milieu de polymérisation contenant un solide catalytique préactivé selon l'invention ne sort nullement du cadre de cette dernière; pareille addition ne conduit toutefois tout au plus qu'à une amélioration de stéréospécificité qui reste marginale.

Lors de l'homopolymérisation du propylène en présence des solides catalytiques préactivés selon l'invention, la proportion de polypropylène amorphe, évaluée en mesurant le poids de polypropylène soluble dans l'heptane bouillant par rapport au polypropylène solide fabriqué au cours de la polymérisation est presque toujours inférieure à 3 %. Cette propriété est conférée au polypropylène solide fabriqué, même lorsque le solide catalytique préactivé a été stocké à haute température (45°C) pendant plusieurs semaines.

Les exemples suivants servent à illustrer l'invention.

Dans ces exemples, les symboles utilisés ont la signification suivante :

I.I = indice d'isotacticité du polymère, apprécié par la fraction de ce dernier, exprimée en % par rapport à la quantité totale de polymère solide recueilli, qui est insoluble dans l'heptane bouillant.

G = module de rigidité en torsion du polymère, mesuré à 100°C et pour un angle de torsion de 60° d'arc, la température du moule étant fixée à 70°C et la durée de conditionnement à 5 minutes (normes BS 2782 - part I - method 150A; ISO 458/l, méthode B; DIN 53447 et ASTM D 1043). Ce module est exprimé en daN/cm$^2$.

MFI = indice de fluidité en fondu mesuré sous une charge de 2,16 kg à 230°C et exprimé en g/10 min. (norme ASTM D 1238).

PSA = poids spécifique apparent de la fraction de polymère insoluble, mesuré par tassement et exprimé en g/l.

$\alpha$ = activité catalytique exprimée conventionnellement en grammes de polymère insoluble dans le milieu de polymérisation, obtenus par heure et par gramme de TiCl$_3$ contenu dans le solide catalytique préactivé.

Exemple 1

A - Préparation du précurseur (solide à base de trichlorure de titane complexé)

Dans un réacteur de 800 ml équipé d'un agitateur à 2 pales tournant à 400 tours/min., on introduit sous atmosphère d'azote 90 ml d'hexane sec et 60 ml de TiCl$_4$ pur. Cette solution hexane-TiCl$_4$ est refroidie à 0 (± 1)°C. Endéans 4 h on y additionne une solution constituée de 190 ml d'hexane et de 70 ml de chlorure de diéthylaluminium (DEAC) en maintenant la température de 0 (± 1)°C dans le réacteur.

Après addition de la solution DEAC-hexane, le milieu de réaction constitué par une suspension de fines particules est maintenu sous agitation à 1 (± 1)°C pendant 15 min., puis est porté en 1 h à 25°C et maintenu 1 h à cette température et ensuite porté en 1 h environ à 65°C. Le milieu est maintenu sous agitation pendant 2 h à 65°C.

La phase liquide est alors séparée du solide et le produit solide est lavé 7 fois au moyen de 200 ml d'hexane sec, avec remise en suspension du solide lors de chaque lavage.

Le solide réduit ainsi obtenu est mis en suspension dans 456 ml de diluant (hexane) et on y ajoute 86 ml d'éther di-isoamylique (EDIA). La suspension est agitée pendant 1 h à 50°C. Ensuite, le solide ainsi traité est séparé de la phase liquide.

Ce dernier solide est remis en suspension dans 210 ml d'hexane et on y ajoute 52 ml de TiCl$_4$; la suspension est maintenue sous agitation (150 tours/min.) à 70°C pendant 2 h. La phase liquide est ensuite éliminée par filtration et le solide à base de trichlorure de titane complexé est lavé 14 fois avec 270 ml d'hexane.

B - Préactivation

Dans un réacteur de 800 ml équipé d'un agitateur à pales tournant à 150 tours/min., on introduit 70 g du solide à base de trichlorure de titane complexé (contenant environ 820 g de TiCl$_3$/kg) suspendu dans 280 ml d'hexane. On introduit lentement (30 minutes) dans ce réacteur 120 ml d'une solution dans l'hexane d'un préactivateur (appelé ci-après préactivateur A) préalablement préparé en mélangeant, par litre d'hexane, 80 g de DEAC (composé (a)) et 176,2 g de 3-(3′,5′-di-tert-butyl-4'-hydroxyphényl)propionate de n-octadécyle commercialisé sous la dénomination Irganox 1076 par CIBA-GEIGY (composé (b)). Le rapport molaire entre les composés (a) et (b) mis en oeuvre pour préparer le préactivateur est donc de 2, et le rapport molaire entre le préactivateur A et le solide à base de trichlorure de titane complexé (exprimé en mole de composé (a) initialement mis en oeuvre par mole de TiCl$_3$ présent dans le solide) vaut 0,2.

La solution de préactivateur n'est introduite dans le réacteur que 15 minutes après le terme du dégagement gazeux observé au cours du mélange du composé (a) et du composé (b).

La suspension ainsi additionnée du préactivateur A est maintenue pendant 1 heure à 30°C sous agitation.

Après décantation, le solide catalytique préactivé résultant est lavé 5 fois au moyen de 100 ml d'hexane sec, avec remise en suspension du solide lors de chaque lavage, puis séché par balayage d'azote en lit fluidisé pendant 2 heures à 70°C.

Le solide catalytique préactivé ainsi obtenu contient, par kg, 641 g de TiCl$_3$, 12 g d'aluminium, 31 g d'EDIA et une quantité, estimée à 250 g environ, de préactivateur A.

C - Polymérisation du propylène en suspension dans le monomère liquide en présence du solide catalytique préactivé

Dans un autoclave de 5 l, préalablement séché et maintenu sous atmosphère d'azote sec, on introduit, sous balayage d'azote :

- 400 mg de DEAC (sous forme d'une solution dans l'hexane à 200 g/l) commercialisé par la firme SCHERING (le rapport a tomique C1/Al est ajusté à 1,02 par addition de dichlorure d'éthylaluminium);
- 100 mg de solide catalytique préactivé (le rapport molaire entre le DEAC et le TiCl$_3$ présent dans le solide vaut ainsi environ 8);
- de l'hydrogène sous pression partielle de 1 bar;
- 3 l de propylène liquide.

On maintient le réacteur à 65°C sous agitation pendant 3 heures. Ensuite, on dégaze le propylène excédentaire et on récupère le polypropylène (PP) formé, soit 643 g de polypropylène sec.

L'activité α du solide catalytique préactivé est 3340; la productivité s'élève à 6430 g de polypropylène/g de solide catalytique préactivé.

Ce polypropylène présente les caractéristiques suivantes :

I.I     = 98,1 %
G       = 678 daN/cm$^2$
MFI     = 3,16 g/10 min.
PSA     = 510 g/l.

Exemples 1R à 5R

Ces exemples sont donnés à titre de comparaison.

Exemple 1R

On prépare un solide à base de trichlorure de titane complexé comme décrit à l'exemple 1, partie A, sans le préactiver comme indiqué à la partie B de cet exemple.

Ce solide, séché comme indiqué à l'exemple 1, contient 811 g de TiCl$_3$, 2,8 g d'aluminium et 61 g d'EDIA.

On effectue un essai de polymérisation en présence du solide non préactivé ainsi obtenu, dans des conditions strictement comparables à celles définies à l'exemple 1, partie C. On récupère, à l'issue de cet essai, 785 g de PP sec.

L'activité α est donc 3230 et la productivité s'élève à 7850 g de PP/g de solide.

Ce polypropylène présente les caractéristiques suivantes :

I.I     = 94,9 %
G       = 572 daN/cm$^2$
MFI     = 7,3 g/10 min.
PSA     = 490 g/l.

Les différences significatives des fractions insolubles dans l'heptane bouillant et des modules G respectifs des polymères obtenus dans des conditions comparables selon les exemples 1 et 1R attestent la stéréospécificité supérieure du système catalytique comprenant le solide catalytique préactivé de l'exemple 1.

Exemple 2R

Un solide à base de trichlorure de titane complexé préparé comme décrit à l'exemple 1, partie A est préactivé par une solution ne contenant que le composé (b). On observe une dissolution partielle du solide qui, par ailleurs, se présente sous forme de grains très fins. L'essai de polymérisation, effectué comme indiqué à l'exemple 1, partie C, est reproduit avec une quantité de catalyseur telle qu'elle contient environ 70 mg de TiCl$_3$.

On obtient 535 g de PP, ce qui correspond à une activité α de 2550 seulement. Ce PP se présente sous la forme de grains fins et son PSA n'est que de 100 g/l, ce qui exclut de pouvoir le mettre en oeuvre.

Exemple 3R

On reproduit l'exemple 1, parties A et B à la seule exception que la suspension de solide à base de trichlorure de titane complexé est additionnée, successivement, d'abord d'une solution dans l'hexane du composé (a), et, 15 minutes après la fin de l'addition de la solution du composé (a), d'une solution dans l'hexane du composé (b). Les valeurs des rapports molaires entre les composés (a) et (b), séparément additionnés, et entre le composé (a) et la quantité de TiCl$_3$ présente dans le solide sont respectivement 2 et 0,2. Le solide catalytique recueilli contient 757 g/kg de TiCl$_3$.

L'essai de polymérisation, effectué comme indiqué à l'exemple 1, partie C, ne permet de recueillir, avec une activité $\alpha$ de 3090, qu'un polypropylène se présentant sous la forme de blocs non manipulables.

Exemple 4R

On reproduit l'exemple 3R, mais en inversant l'ordre d'introduction des solutions de composés (a) et (b). On observe le même phénomène qu'à l'exemple 2R, c'est-à-dire une dissolution partielle du solide.

L'essai de polymérisation, effectué comme décrit à l'exemple 1, partie C, ne permet de recueillir, avec une activité $\alpha$ de 3450, qu'un polypropylène qui se présente sous la forme de très fins grains, dont le PSA n'est que de 200 g/l, ce qui exclut de pouvoir le mettre en oeuvre.

Exemple 5R

Le solide à base de trichlorure de titane complexé préparé comme décrit à l'exemple 1R (c'est-à-dire non préactivé) est mis en oeuvre dans un essai de polymérisation réalisé comme décrit à l'exemple 1, partie C, à cette exception que l'on introduit dans le milieu de polymérisation, outre le DEAC, le solide, l'hydrogène et le propylène, le produit Irganox 1076 en quantité telle que le rapport molaire entre ce produit et le TiCl$_3$ présent dans le solide soit d'environ 0,2.

On obtient, avec une activité $\alpha$ de 3286, un PP caractérisé par les propriétés ci-après :

I.I = 95,2 %
G = 575 daN/cm$^2$
MFI = 5,2 g/10 min.
PSA = 505 g/l.

Exemple 2

On prépare un solide catalytique préactivé comme indiqué à l'exemple 1, parties A et B, sauf que l'on remplace le produit Irganox 1076 par du 2,6-di-tert-butyl-4-méthyl-phénol commercialisé sous la dénomination Ionol CP par SHELL.

Le solide catalytique préactivé ainsi obtenu contient par kg, 632 g de TiCl$_3$, 14 g d'aluminium, 30 g d'EDIA et une quantité de préactivateur estimée à 170 g environ. Il est utilisé pour effectuer un essai de polymérisation dans les conditions de l'exemple 1, partie C.

Cet essai permet de recueillir, avec une activité $\alpha$ de 3230, un polypropylène présentant les caractéristiques suivantes :

I.I = 95,9 %
G = 653 daN/cm$^2$
MFI = 9 g/10 min.
PSA = 500 g/l.

Exemple 3

Un solide catalytique préactivé préparé comme indiqué à l'exemple 1, parties A et B, est utilisé dans un essai de polymérisation du propylène en suspension dans l'hexane dans les conditions opératoires décrites ci-après.

Dans un autoclave de 5 litres en acier inoxydable et purgé plusieurs fois à l'aide d'azote, on introduit 1 litre d'hexane sec et épuré. On introduit ensuite successivement 400 mg de DEAC (sous la forme d'une solution dans l'hexane à 200 g/l) et une quantité de solide catalytique équivalant à environ 51 mg de TiCl$_3$. Le rapport molaire DEAC/TiCl$_3$ est alors d'environ 10.

L'autoclave est chauffé à 65°C et est remis à pression atmosphérique par un dégazage lent. Ensuite, on y réalise une pression absolue d'hydrogène de 0,3 bar; puis on introduit dans l'autoclave du propylène jusqu'à atteindre une pression totale à la température considérée de 11,9 bars. Cette pression est maintenue constante pendant la polymérisation par l'introduction de propylène gazeux.

Après 3 h, on arrête la polymérisation par dégazage du propylène.

Le contenu de l'autoclave est versé sur un filtre Büchner, rincé trois fois par 0,5 l d'hexane et séché sous pression réduite à 60°C. On recueille 251 g de PP insoluble dans l'hexane. Dans l'hexane de polymérisation et de lavage, on trouve 0,75 g de polymère soluble, ce qui correspond à 0,3 %. L'activité $\alpha$

est de 1643. La productivité s'élève à 3157 g de PP/g de solide catalytique préactivé.

Le PP insoluble dans l'hexane présente les propriétés suivantes :

I.I     = 98,2 %
G       = 654 daN/cm$^2$
MFI     = 2,9 g/10 min.
PSA     = 503 g/l.

Exemple 6R

Cet exemple est donné à titre de comparaison.

On effectue, en présence d'un solide catalytique préparé comme indiqué à l'exemple 3, mais en omettant l'étape de préactivation, et qui contient 735 g/kg de TiCl$_3$, un essai de polymérisation dans les mêmes conditions qu'à l'exemple 3. On obtient, avec une activité α de 1719, un PP, dont 1 % est soluble dans l'hexane de polymérisation et de lavage et dont la partie insoluble présente les caractéristiques suivantes :

I.I     = 95,7 %
G       = 591 daN/cm$^2$
MFI     = 9,5 g/10 min.
PSA     = 479 g/l.

Exemple 4

On prépare un solide catalytique préactivé selon les conditions générales exposées à l'exemple 1, parties A et B. Toutefois, après le traitement de la suspension de solide réduit sous agitation pendant 2 heures à 65°C, cette suspension est refroidie à environ 55°C; on introduit alors, dans le ciel gazeux du réacteur, du propylène sous une pression de 2 bars. Cette introduction est poursuivie pendant le temps suffisant (environ 45 minutes) pour obtenir, par kg de solide, 100 g de propylène polymérisé. La suspension du solide ainsi "prépolymérisé" est ensuite refroidie à 40°C et la préparation est ensuite poursuivie comme indiqué à l'exemple 1, partie A.

Le solide catalytique préactivé finalement obtenu contient par kg 611 g de TiCl$_3$, 9 g d'aluminium, 14 g d'EDIA et une quantité, estimée à 143 g environ, de préactivateur A.

Ce solide catalytique préactivé est mis en oeuvre dans un essai de polymérisation du propylène comprenant une première étape effectuée dans le monomère liquide et une seconde étape effectuée en phase gazeuse dans les conditions opératoires détaillées ci-après.

Dans l'autoclave de 5 l utilisé selon les exemples 1 et 3, on introduit sous courant d'azote :
- 800 mg de DEAC
- une quantité de solide catalytique équivalant à 100 mg de TiCl$_3$.
Le rapport molaire DEAC/TiCl$_3$ est alors d'environ 10.

On réalise dans l'autoclave une pression absolue d'hydrogène de 0,2 bar. Puis, on introduit sous agitation 2 l de propylène liquide et on chauffe l'autoclave à 60°C. On polymérise 30 min. à cette température. On dégaze l'autoclave ensuite à une pression de 15 bars en le chauffant en même temps à 70°C. On y réalise ensuite une pression absolue d'hydrogène de 1 bar, puis on introduit du propylène dans l'autoclave jusqu'à atteindre une pression totale à la température considérée de 28 bars. Après 3 heures, on arrête la polymérisation par dégazage du propylène et on récupère le PP formé, soit 1150 g de PP sec.

L'activité α du solide catalytique préactivé est donc 3286 et la productivité s'élève à 7027 g de PP/g de solide catalytique préactivé. Ce PP présente les caractéristiques suivantes :

I.I     = 97,9 %
G       = 698 daN/cm$^2$
MFI     = 3 g/10 min.
PSA     = 520 g/l.

Exemple 7R

Cet exemple est donné à titre de comparaison.

On prépare un solide catalytique préactivé selon les indications de l'exemple 4, mais en omettant l'étape de préactivation. Ce solide contient, par kg, 718 g de TiCl$_3$, 3,8 g d'aluminium et 84 g d'EDIA. Utilisé dans un essai de polymérisation effectué selon les conditions exposées à l'exemple 4, il permet d'obtenir, avec une activité α de 3168, un PP caractérisé par les propriétés suivantes :

I.I     = 96,4 %
G       = 620 daN/cm$^2$
MFI    = 3 g/10 min.
PSA   = 516 g/l.

Exemples 5 à 7

On prépare des solides catalytiques préactivés selon les conditions mentionnées à l'exemple 4, sauf que l'on modifie le rapport molaire entre les composés (a) et (b) mis en oeuvre pour préparer le préactivateur A (voir exemple 1, partie B) (exemples 5 et 6) ainsi que le rapport molaire entre le préactivateur A et le solide à base de trichlorure de titane complexé (exprimé en mole de composé (a) initialement mis en oeuvre par mole de TiCl$_3$ présent dans le solide) (voir exemple 1, idem) (exemple 7).

Ces solides catalytiques préactivés sont utilisés dans des essais de polymérisation du propylène en suspension dans l'hexane selon les conditions générales mentionnées à l'exemple 3.

Les conditions opératoires particulières des préparations des solides catalytiques et les résultats des essais de polymérisation sont rassemblés dans le tableau I ci-après.

Tableau I

| Exemple | 5 | 6 | 7 |
|---|---|---|---|
| Préparation des solides catalytiques préactivés | | | |
| $\dfrac{\text{composé (a)}}{\text{composé (b)}}$ (mole/mole) | 50 | 10 | 10 |
| $\dfrac{\text{composé (a)}}{\text{TiCl}_3 \text{ contenu dans le solide}}$ (mole/mole) | 1 | 1 | 0,2 |
| Teneur du solide catalytique préactivé en $TiCl_3$ (g/kg) | 724 | 672 | 709 |
| Résultats de polymérisation | | | |
| Activité $\alpha$ (g PP/g $TiCl_3$ x h) | 2160 | 2160 | 2130 |
| PP soluble dans l'hexane de polymérisation (en % de PP total) | 1,1 | 0,8 | 1,1 |
| I.I (%) | 97,3 | 98,2 | 97,4 |
| G (daN/cm$^2$) | 678 | 688 | 689 |
| MFI (g/10 min.) | 7,1 | 5,0 | 7,7 |
| PSA (g/l) | 502 | 502 | 504 |

Exemple 8

On prépare un solide catalytique préactivé selon les indications de l'exemple 4 et on l'utilise dans un essai de polymérisation effectué selon les conditions exposées à la partie C de l'exemple 1 sauf que l'on maintient le réacteur à 75° C sous agitation pendant 2 heures.

14

Dans ces conditions, ce solide catalytique préactivé permet d'obtenir avec un activité $\alpha$ de 5010, un PP caractérisé par les propriétés suivantes

| | |
|---|---|
| I.I | = 98,1 % |
| G | = 688 daN/cm$^2$ |
| MFI | = 5,9 g/10 min. |
| PSA | = 510 g/l. |

Exemples 9 et 10

On prépare des solides catalytiques préactivés selon les conditions mentionnées à l'exemple 1, parties A et B sauf que l'on utilise, comme composés (a), respectivement, du triéthylaluminium (TEAL) (exemple 9); du dichlorure d'éthylaluminium (EADC) (exemple 10).

Ces solides catalytiques préactivés sont utilisés dans des essais de polymérisation du propylène, en suspension dans le monomère liquide, dans les conditions générales mentionnées à l'exemple 1, partie C.

Les particularités des solides catalytiques préactivés utilisés et les résultats de ces essais de polymérisation sont rassemblés dans le tableau II ci-après.

### Tableau II

| Exemple | 9 | 10 |
|---|---|---|
| Nature du composé (a) utilisé pour préparer le solide catalytique préactivé | TEAL | EADC |
| Teneur du solide catalytique préactivé en TiCl$_3$ (g/kg) | 648 | 713 |
| Activité $\alpha$ (g PP/g TiCl$_3$ x h) | 3136 | 2875 |
| Propriétés du PP | | |
| I.I (%) | 98,0 | 97,7 |
| G (daN/cm$^2$) | 645 | 667 |
| MFI (g/10 min.) | 3,4 | 3,7 |
| PSA (g/l) | 504 | 490 |

## Revendications

1. Solide catalytique à base de trichlorure de titane complexé, utilisable pour la polymérisation stéréospécifique des alpha-oléfines, qui a été préactivé par mise en contact avec un préactivateur organoalumini-

EP 0 261 727 B1

que et séparé de son milieu de préactivation, caractérisé en ce que ledit préactivateur comprend le produit de la réaction d'un composé (a) choisi parmi les composés organoaluminiques et d'un composé (b) choisi parmi les composés hydroxyaromatiques dont le groupe hydroxyle est stériquement bloqué.

2. Solide suivant la revendication 1, caractérisé en ce que le composé (a) est choisi parmi les composés de formule :

$$Al\ R_n\ X_{3-n}$$

dans laquelle :
- R représente des radicaux hydrocarbonés identiques ou différents contenant de 1 à 18 atomes de carbone,
- X est un halogène,
- n est un nombre tel que $0 < n \leq 3$.

3. Solide suivant les revendications 1 et 2, caractérisé en ce que le composé (a) est choisi parmi les trialkylaluminiums et les halogénures de dialkylaluminium.

4. Solide suivant la revendication 1, caractérisé en ce que le composé (b) est choisi parmi les hydroxyarylènes mono- ou polycycliques comprenant un radical alkyle secondaire ou tertiaire dans les deux positions ortho par rapport au groupe hydroxyle.

5. Solide suivant les revendications 1 et 4, caractérisé en ce que le composé (b) est choisi parmi les monophénols monocycliques di-tert-alkylés et les monoesters de l'acide 3-(3',5'-di-tert-butyl-4'-hydroxyphényl)propionique.

6. Solide suivant les revendications 1 à 5, caractérisé en ce que le préactivateur est préparé en mettant en présence le composé (a) et le composé (b) selon un rapport molaire compris entre 50 et 0,1 moles de composé (a) par mole de composé (b).

7. Solide suivant la revendication 6, caractérisé en ce que le préactivateur est préparé en mettant en présence un composé (a) choisi parmi les chlorures de dialkylaluminium, et un composé (b) choisi parmi les monoesters de l'acide 3-(3',5'-di-tert-butyl-4'-hydroxyphényl)propionique.

8. Solide suivant les revendications 1 à 6, caractérisé en ce que le produit de la réaction du composé (a) avec le composé (b) répond à la formule empirique :

$$R_p\ Al(OR')_q\ X_{3-(p+q)}$$

dans laquelle :
- R représente le(les) même(s) radical(radicaux) hydrocarboné(s) que celui(ceux) contenu(s) dans le composé (a);
- OR' représente le groupe aryloxy dérivé du composé (b);
- X est l'halogène éventuellement contenu dans le composé (a);
- p est un nombre tel que $0 < p < 3$;
- q est un nombre tel que $0 < q < 2$;
- la somme (p + q) est telle que $0 < (p + q) \leq 3$.

9. Solide suivant les revendications 1 à 8, caractérisé en ce que la mise en contact avec le préactivateur se fait par introduction du préactivateur dans une suspension du solide à préactiver dans un diluant hydrocarboné inerte.

10. Solide suivant les revendications 1 à 9, caractérisé en ce que le préactivateur est mis en contact avec le solide à préactiver selon un rapport molaire entre la quantité initiale totale de composé (a) mise en oeuvre et la quantité de $TiCl_3$ contenue dans le solide compris entre $10^{-2}$ et 1 mole/mole.

11. Solide suivant les revendications 1 à 10, caractérisé en ce qu'il a été préactivé par mise en contact du préactivateur avec un précurseur solide répondant à la formule :

16

$TiCl_3.(AlR''Cl_2)_x.C_y$

où R'' est un radical alkyle comprenant de 2 à 6 atomes de carbone, C est un agent complexant choisi parmi les éthers aliphatiques dont les radicaux aliphatiques comprennent de 2 à 8 atomes de carbone, x est un nombre quelconque inférieur à 0,20 et y est un nombre quelconque supérieur à 0,009.

12. Solide suivant la revendication 11, caractérisé en ce que le précurseur solide se présente sous la forme de particules sphériques de diamètre compris entre 5 et 100 microns, constituées d'un aggloméret de microparticules également sphériques qui ont un diamètre compris entre 0,05 et 1 micron et dont la porosité est telle que la surface spécifique du précurseur solide est compris entre 100 et 250 $m^2/g$ et que sa porosité interne totale est comprise entre 0,15 et 0,35 $cm^3/g$.

13. Procédé pour la préparation d'un solide catalytique à base de trichlorure de titane complexé utilisable pour la polymérisation stéréospécifique des alpha-oléfines, préactivé par mise en contact d'un précurseur solide à base de trichlorure de titane complexé avec un préactivateur organoaluminique et séparation du solide préactivé de son milieu de préactivation, caractérisé en ce que ledit préactivateur comprend le produit de la réaction d'un composé (a) choisi parmi les composés organoaluminiques et d'un composé (b) choisi parmi les composés hydroxyaromatiques dont le groupe hydroxyle est stériquement bloqué.

14. Procédé suivant la revendication 13, caractérisé en ce que le composé (a) est choisi parmi les composés de formule :

$Al R_n X_{3-n}$

dans laquelle :
   - R représente des radicaux hydrocarbonés identiques ou différents contenant de 1 à 18 atomes de carbone,
   - X est un halogène,
   - n est un nombre tel que $0 < n \leqq 3$.

15. Procédé suivant la revendication 13, caractérisé en ce que le composé (b) est choisi parmi les hydroxyarylènes mono- ou polycycliques comprenant un radical alkyle secondaire ou tertiaire dans les deux positions ortho par rapport au groupe hydroxyle.

16. Procédé suivant les revendications 13 à 15, caractérisé en ce que le préactivateur est préparé en mettant en présence le composé (a) et le composé (b) selon un rapport molaire compris entre 50 et 0,1 moles de composé (a) par mole de composé (b).

17. Procédé suivant les revendications 13 à 16, caractérisé en ce que le préactivateur est mis en contact avec le précurseur solide selon un rapport molaire entre la quantité initiale totale de composé (a) mise en oeuvre et la quantité de $TiCl_3$ contenue dans le précurseur compris entre $10^{-2}$ et 1 mole/mole.

18. Procédé suivant les revendications 13 à 17, caractérisé en ce que la mise en contact du précurseur solide avec le préactivateur se fait par introduction du préactivateur dans une suspension du précurseur dans un diluant hydrocarboné inerte.

19. Procédé suivant les revendications 13 à 18, caractérisé en ce que le précurseur solide et le préactivateur sont maintenus en contact à une température comprise entre 20 et 40°C pour une durée comprise entre 15 et 90 minutes.

20. Procédé suivant les revendications 13 à 19, caractérisé en ce que le solide catalytique préactivé est lavé par un diluant hydrocarboné inerte avant d'être utilisé en polymérisation.

21. Procédé pour la polymérisation d'alpha-oléfines en présence d'un système catalytique comprenant un activateur choisi parmi les composés organométalliques de métaux des groupes Ia, IIa, IIb et IIIb du Tableau Périodique et un solide catalytique à base de trichlorure de titane complexé et préactivé, caractérisé en ce qu'on met en oeuvre un solide catalytique conforme à l'une quelconque des revendications 1 à 12.

**22.** Procédé suivant la revendication 21, appliqué à la polymérisation stéréospécifique du propylène.

**23.** Procédé suivant la revendication 21, appliqué à la polymérisation stéréospécifique du propylène en suspension dans un diluant hydrocarboné inerte.

**24.** Procédé suivant la revendication 21, appliqué à la polymérisation stéréospécifique du propylène dans le monomère à l'état liquide.

**25.** Procédé suivant la revendication 21, appliqué à la polymérisation stéréospécifique du propylène en phase gazeuse.

**Claims**

**1.** Complexed titanium trichloride-based catalytic solid which can be used for the stereospecific polymerisation of alpha-olefins, which has been preactivated by bringing it into contact with an organoaluminium preactivator and separated from its preactivation medium, characterised in that the said preactivator comprises the product of reaction between a compound (a) chosen from among organoaluminium compounds and a compound (b) chosen from among hydroxyaromatic compounds in which the hydroxyl group is sterically hindered.

**2.** Solid according to Claim 1, characterised in that the compound (a) is chosen from among compounds of formula:

$$AlR_nX_{3-n}$$

in which:
- R represents hydrocarbon radicals, which may be identical or different, containing from 1 to 18 carbon atoms,
- X is a halogen, and
- n is a number such that $0 < n \leq 3$.

**3.** Solid according to Claims 1 and 2, characterised in that the compound (a) is chosen from among trialkylaluminiums and dialkylaluminium halides.

**4.** Solid according to Claim 1, characterised in that the compound (b) is chosen from among mono- or polycyclic hydroxyarylenes containing a secondary or tertiary alkyl radical in the two ortho positions relative to the hydroxyl group.

**5.** Solid according to Claims 1 and 4, characterised in that the compound (b) is chosen from among di-tert-alkylated monocyclic monophenols and 3-(3',5'-di-tert-butyl-4'-hydroxyphenyl)propionic acid monoesters.

**6.** Solid according to Claims 1 to 5, characterised in that the preactivator is prepared by bringing compound (a) and compound (b) into contact with each other in a molar ratio of between 50 and 0.1 moles of compound (a) per mole of compound (b).

**7.** Solid according to Claim 6, characterised in that the preactivator is prepared by bringing into contact with each other a compound (a) chosen from among dialkylaluminium chlorides and a compound (b) chosen from among 3-(3',5'-di-tert-butyl-4'-hydroxyphenyl)propionic acid monoesters.

**8.** Solid according to Claims 1 to 6, characterised in that the product of reaction between compound (a) and compound (b) corresponds to the empirical formula:

$$R_pAl(OR')_qX_{3-(p+q)}$$

18

in which:
- R represents the same hydrocarbon radical(s) as that (those) contained in compound (a);
- OR' represents the aryloxy group derived from compound (b);
- X is the halogen optionally contained in compound (a);
- p is a number such that $0 < p < 3$;
- q is a number such that $0 < q < 2$; and
- the sum $(p + q)$ is such that $0 < (p + q) \leq 3$.

9. Solid according to Claims 1 to 8, characterised in that the contacting with the preactivator is carried out by introducing the preactivator into a suspension of the solid to be preactivated in an inert hydrocarbon diluent.

10. Solid according to Claims 1 to 9, characterised in that the preactivator is brought into contact with the solid to be preactivated in a molar ratio of the total initial quantity of compound (a) employed to the quantity of $TiCl_3$ contained in the solid of between $10^{-2}$ and 1 mole/mole.

11. Solid according to Claims 1 to 10, characterised in that it has been preactivated by bringing the preactivator into contact with a solid precursor corresponding to the formula:

$$TiCl_3.(AlR''Cl_2)_x.C_y$$

in which R'' is an alkyl radical containing from 2 to 6 carbon atoms, C is a complexing agent chosen from among aliphatic ethers in which the aliphatic radicals contain from 2 to 8 carbon atoms, x is any number smaller than 0.20 and y is any number greater than 0.009.

12. Solid according to Claim 11, characterized in that the solid precursor is in the form of spherical particles of diameter between 5 and 100 microns, which consist of an agglomerate of microparticles which are also spherical and which have a diameter of between 0.05 and 1 micron and whose porosity is such that the specific surface area of the solid precursor is between 100 and 250 $m^2$/g and that its total internal porosity is between 0.15 and 0.35 $cm^3$/g.

13. Process for the preparation of a complexed titanium trichloride-based catalytic solid which can be used for the stereospecific polymerisation of alpha-olefins, preactivated by bringing a complexed titanium trichloride-based solid precursor into contact with an organoaluminium preactivator and separation of the preactivated solid from its preactivation medium, characterised in that the said preactivator comprises the product of reaction between a compound (a) chosen from among organoaluminium compounds and a compound (b) chosen from among hydroxyaromatic compounds in which the hydroxyl group is sterically hindered.

14. Process according to Claim 13, characterised in that the compound (a) is chosen from among compounds of formula:

$$AlR_nX_{3-n}$$

in which:
- R represents hydrocarbon radicals, which may be identical or different, containing from 1 to 18 carbon atoms,
- X is a halogen, and
- n is a number such that $0 < n \leq 3$.

15. Process according to Claim 13, characterised in that the compound (b) is chosen from among mono- or polycyclic hydroxyarylenes containing a secondary or tertiary alkyl radical in the two ortho positions relative to the hydroxyl group.

16. Process according to Claims 13 to 15, characterised in that the preactivator is prepared by bringing compound (a) and compound (b) into contact with each other in a molar ratio of between 50 and 0.1 moles of compound (a) per mole of compound (b).

**17.** Process according to Claims 13 to 16, characterised in that the preactivator is brought into contact with the solid precursor in a molar ratio of the total initial quantity of compound (a) employed to the quantity of $TiCl_3$ contained in the precursor of between $10^{-2}$ and 1 mole/mole.

**18.** Process according to Claims 13 to 17, characterised in that the contacting of the solid precursor with the preactivator is carried out by introducing the preactivator into a suspension of the precursor in an inert hydrocarbon diluent.

**19.** Process according to Claims 13 to 18, characterised in that the solid precursor and the preactivator are maintained in contact with each other at a temperature between 20 and 40°C for a period between 15 and 90 minutes.

**20.** Process according to Claims 13 to 19, characterised in that the preactivated catalytic solid is washed with an inert hydrocarbon diluent before being used in polymerisation.

**21.** Process for the polymerisation of alpha-olefins in the presence of a catalytic system comprising an activator chosen from among organometallic compounds of metals of groups Ia, IIa, IIb and IIIb of the Periodic Table and a complexed and preactivated titanium trichloride-based catalytic solid, characterised in that a catalytic solid according to any of Claims 1 to 12 is employed.

**22.** Process according to Claim 21, applied to the stereospecific polymerisation of propylene.

**23.** Process according to Claim 21, applied to the stereospecific polymerisation of propylene in suspension in an inert hydrocarbon diluent.

**24.** Process according to Claim 21, applied to the stereospecific polymerisation of propylene in the monomer in the liquid state.

**25.** Process according to Claim 21, applied to the stereospecific polymerisation of propylene in the gaseous phase.

**Patentansprüche**

**1.** Katalytischer fester Stoff auf der Basis von komplexiertem Titantrichlorid verwendbar bei der stereospezifischen Polymerisation von Alpha-Olefinen, der durch Kontaktieren mit einem Organoaluminium-Preaktivator preaktiviert und aus seinem Preaktivierungsmedium getrennt wurde, dadurch gekennzeichnet, daß der Preaktivator das Reaktionsprodukt einer Verbindung (a), ausgewählt unter den Organoaluminiumverbindungen, und einer Verbindung (b), ausgewählt unter den hydroxyaromatischen Verbindungen, deren Hydroxylgruppe sterisch blockiert ist, umfaßt.

**2.** Fester Stoff gemäß Anspruch 1, dadurch gekennzeichnet, daß die Verbindung (a) ausgewählt ist unter den Verbindungen der Formel:

$$Al\ R_n\ X_{3-n}$$

in der:
- R identische oder unterschiedliche, Kohlenwasserstoff-Reste mit 1 bis 18 Kohlenstoffatomen darstellt,
- X ein Halogen ist,
- n eine Zahl entsprechend $0 < n \leqq 3$ ist.

**3.** Fester Stoff gemäß den Ansprüchen 1 und 2, dadurch gekennzeichnet, daß die Verbindung (a) ausgewählt ist unter den Trialkylaluminium-Verbindungen und den Dialkylaluminium-Halogeniden.

**4.** Fester Stoff gemäß Anspruch 1, dadurch gekennzeichnet, daß die Verbindung (b) ausgewählt ist unter den mono- oder polyzyklischen Hydroxyarylenen, die einen sekundären oder tertiären Alkylrest in den beiden Ortho-Positionen bezüglich der Hydroxylgruppe umfassen.

20

**5.** Fester Stoff gemäß den Ansprüchen 1 und 4, dadurch gekennzeichnet, daß die Verbindung (b) ausgewählt ist unter den monozyklischen Di-tert-alkyl-Monophenolen und den Monoestern der 3-(3',5'-di-tert-butyl-4'-hydroxyphenyl)Propionsäure.

**6.** Fester Stoff gemäß den Ansprüchen 1 bis 5, dadurch gekennzeichnet, daß der Preaktivator hergestellt wird, durch Vorlegen der Verbindung (a) und der Verbindung (b) gemäß einem molaren Verhältnis zwischen 50 und 0,1 Mol von Verbindung (a) pro Mol von Verbindung (b).

**7.** Fester Stoff gemäß Anspruch 6, dadurch gekennzeichnet, daß der Preaktivator hergestellt ist durch Vorlegen einer Verbindung (a), ausgewählt unter den Dialkylaluminiumchloriden, und einer Verbindung (b), ausgewählt unter den Monoestern der 3-(3',5,-di-tert-butyl-4'-hydroxyphenyl)Propionsäure.

**8.** Fester Stoff gemäß den Ansprüchen 1 bis 6, dadurch gekennzeichnet, daß das Reaktionsprodukt der Verbindung (a) mit der Verbindung (b) der empirischen Formel:

$$R_p \, Al(OR')_q \, X_{3-(p+q)}$$

entspricht, in der
- R den(die) gleichen Kohlenwasserstoff-Rest(e), wie den(die) in der Verbindung (a) enthaltenen darstellt:
- OR' die von der Verbindung (b) abgeleitete Aryloxygruppe darstellt:
- X das gegebenenfalls in der Verbindung (a) enthaltene Halogen ist;
- p eine Zahl entsprechend $0 < p < 3$ ist;
- q eine Zahl entsprechend $0 < q < 2$ ist;
- die Summe $(p+q)$ entsprechend $0 < (p+q) \leq 3$ ist.

**9.** Fester Stoff gemäß den Ansprüchen 1 bis 8, dadurch gekennzeichnet, daß das Kontaktieren mit dem Preaktivator durch Einführen des Preaktivators in eine Suspension des zu preaktivierenden festen Stoffes in einem inerten, Kohlenwasserstoff-Verdünnungsmittel, durchgeführt wird.

**10.** Fester Stoff gemäß den Ansprüchen 1 bis 9, dadurch gekennzeichnet, daß der Preaktivator mit dem zu preaktivierenden festen Stoff in Kontakt gebracht wird, gemäß einem molaren Verhältnis zwischen der gesamten, eingesetzten Ausgangsmenge von Verbindung (a) und der in dem festen Stoff enthaltenen Menge an $TiCl_3$ zwischen $10^{-2}$ und 1 Mol/Mol.

**11.** Fester Stoff gemäß den Ansprüchen 1 bis 10, dadurch gekennzeichnet, daß er preaktiviert wurde durch Kontaktieren des Preaktivators mit einem festen Vorläufer, der der Formel:

$$TiCl_3 . (AlR''Cl_2)_x . C_y$$

entspricht, in der R'' ein Alkylrest mit 2 bis 6 Kohlenstoffatomen, C ein Komplexbildner, ausgewählt unter den aliphatischen Ethern, deren aliphatischen Reste 2 bis 8 Kohlenstoffatome umfassen, x eine Zahl kleiner als 0,20 und y eine Zahl größer als 0,009 ist.

**12.** Fester Stoff gemäß Anspruch 11, dadurch gekennzeichnet, daß der feste Vorläufer sich darstellt in Form von sphärischen Teilchen mit einem Durchmesser zwischen 5 und 100 Mikron, die aus einem Agglomerat von ebenfalls sphärischen Mikroteilchen mit einem Durchmesser zwischen 0,05 und 1 Mikron gebildet sind und deren Porosität derart ist, daß die spezifische Oberfläche des festen Vorläufers zwischen 100 und 250 $m^2/g$ umfaßt und dessen gesamte innere Porosität zwischen 0,15 und 0,35 $cm^3/g$ umfaßt.

**13.** Verfahren zur Herstellung eines katalytischen festen Stoffes auf der Basis von komplexiertem Titantrichlorid verwendbar bei der stereospezifischen Polymerisation von Alpha-Olefinen, der durch Kontaktieren eines festen Vorläufers auf der Basis von komplexiertem Titantrichlorid mit einem Organoaluminium-Preaktivator preaktiviert wurde und Trennen des preaktivierten festen Stoffes aus seinem Preaktivierungsmedium, dadurch gekennzeichnet, daß der Preaktivator das Reaktionsprodukt einer Verbindung (a), ausgewählt unter den Organoaluminiumverbindungen, und einer Verbindung (b), ausgewählt unter den hydroxyaromatischen Verbindungen, deren Hydroxylgruppe sterisch blockiert ist, umfaßt.

**14.** Verfahren gemäß Anspruch 13, dadurch gekennzeichnet, daß die Verbindung (a) ausgewählt ist unter den Verbindungen der Formel:

$$Al\ R_n\ X_{3-n}$$

in der:
- R identische oder unterschiedliche Kohlenwasserstoff-Reste mit 1 bis 18 Kohlenstoffatomen darstellt,
- X ein Halogen ist,
- n ein Zahl entsprechend $0 < n \leq 3$ ist.

**15.** Verfahren gemäß Anspruch 13, dadurch gekennzeichnet, daß die Verbindung (b) ausgewählt ist unter den mono- oder polyzyklischen Hydroxyarylenen, die einen sekundären oder tertiären Alkylrest in den beiden Ortho-Positionen bezüglich der Hydroxylgruppe umfassen.

**16.** Verfahren gemäß den Ansprüchen 13 bis 15, dadurch gekennzeichnet, daß der Preaktivator hergestellt wird, durch Vorlegen der Verbindung (a) und der Verbindung (b) gemäß einem molaren Verhältnis zwischen 50 und 0,1 Mol von Verbindung (a) pro Mol von Verbindung (b).

**17.** Verfahren gemäß den Ansprüchen 13 bis 16, dadurch gekennzeichnet, daß der Preaktivator mit dem festen Vorläufer in Kontakt gebracht wird, gemäß einem molaren Verhältnis zwischen der gesamten, eingesetzten Ausgangsmenge von Verbindung (a) und der in dem Vorläufer enthaltenen Menge an TiCl$_3$, zwischen $10^{-2}$ und 1 Mol/Mol.

**18.** Verfahren gemäß den Ansprüchen 13 bis 17, dadurch gekennzeichnet, daß das Kontaktieren des festen Vorläufers mit dem Preaktivator durch Einführen des Preaktivators in eine Suspension des Vorläufers in einem inerten, Kohlenwasserstoff-Verdünnungsmittel durchgeführt wird.

**19.** Verfahren gemäß den Ansprüchen 13 bis 18, dadurch gekennzeichnet, daß der feste Vorläufer und der Preaktivator bei einer Temperatur zwischen 20 und 40°C während einer Dauer von 15 bis 90 Minuten in Kontakt gehalten werden.

**20.** Verfahren gemäß den Ansprüchen 13 bis 19, dadurch gekennzeichnet, daß der preaktivierte katalytische feste Stoff durch ein inertes, Kohlenwasserstoff-Verdünnungsmittel gewaschen wird, bevor er bei der Polymerisation verwendet wird.

**21.** Verfahren zur Polymerisation von Alpha-Olefinen in Anwesenheit eines katalytischen Systems, das einen Aktivator, ausgewählt unter den organometallischen Verbindungen von Metallen der Gruppen Ia, IIa, IIb und IIIb der Tabelle des Periodischen Systems und einen katalytischen festen Stoff auf der Basis von komplexiertem und preaktiviertem Titantrichlorid umfaßt, dadurch gekennzeichnet, daß ein katalytischer fester Stoff gemäß einem der Ansprüche 1 bis 12 eingesetzt wird.

**22.** Verfahren gemäß Anspruch 21 zur Verwendung bei der stereospezifischen Polymerisation des Propylens.

**23.** Verfahren gemäß Anspruch 21 zur Verwendung bei der stereospezifischen Polymerisation des Propylens in Suspension in einem inerten, Kohlenwasserstoff-Verdünnungsmittel.

**24.** Verfahren gemäß Anspruch 21 zur Verwendung bei der stereospezifischen Polymerisation des Propylens in dem Monomer in flüssigem Zustand.

**25.** Verfahren gemäß Anspruch 21 zur Verwendung bei der stereospezifischen Polymerisation des Propylens in gasförmiger Phase.